# EUROPEAN PATENT SPECIFICATION

(11) **EP 2 852 443 B1**
(45) Date of publication and mention of the grant of the patent: **03.10.2018**
(21) Application number: 13724691.4
(22) Date of filing: 09.05.2013
(51) Int. Cl.: A63B 71/00, A63B 21/06, A63B 21/065, A63B 21/00, A63B 23/20, A61F 5/41

(54) **EXERCISE DEVICE AND METHOD**
ÜBUNGSVORRICHTUNG UND -VERFAHREN
DISPOSITIF D'EXERCICE ET PROCÉDÉ ASSOCIÉ

(30) Priority: 10.05.2012 US 201213468840
(43) Date of publication of application: 01.04.2015
(73) Proprietor: Mandell, David, Chevy Chase, MD 20815 (US)
(72) Inventor: Mandell, David, Chevy Chase, MD 20815 (US)
(74) Representative: HGF Limited
(86) International application number: PCT/US2013/040370
(87) International publication number: WO 2013/170046

(56) References cited:
- WO-A2-2010/131252
- CA-A1- 2 310 002
- CN-Y- 2 707 316
- US-A- 5 060 936
- US-A- 5 267 927
- US-A- 5 702 330
- US-A1- 2002 153 014
- US-A1- 2006 270 533

## Description

### BACKGROUND OF THE INVENTION

The present invention relates to exercise devices for enhanced sexual function and for the prevention of incontinence, and more particularly, to a device and method for performing male pelvic muscle exercises, also known as "Kegel exercises."

The muscles surrounding the base of the penis, anus and prostate gland are known as the pubococcygeus muscles. Exercises of the pubococcygeus muscles were developed by Dr. Arnold Kegel in the mid-1940's to prepare women for the physiological stresses of the late stages of pregnancy and vaginal childbirth. It was later discovered that the exercises, when performed by men, provided significant benefits.

For men, exercising the pubococcygeus muscles can create harder and firmer erections, improve sexual performance, heighten orgasms, aid in reducing premature ejaculation and improve prostate health. Additionally, there is evidence that exercising the pubococcygeus muscles may help prevent and treat urinary incontinence and post void dribbling.

For men, Kegel exercises typically consist of a series of motions in which the individual squeezes and releases his pubococcygeus muscles. Dr. Kegel, in his research, however, also noted that adding resistance to these exercises further developed and strengthened the pubococcygeus muscles and could lead to more significant results. Typically, Kegel exercises are performed by an individual in private without a device that provides resistance.
CN 2 707 316 Y relates to an apparatus for male health care, which comprises a penis coating device and a gravity object; the penis coating device is connected with the gravity object.

Prior attempts at devices for providing resistance for Kegel exercises suffer from at least one of the following shortcomings: the devices are cumbersome and difficult to use, the devices pose a risk of injury to a user, the devices do not allow the user to provide assistance to himself during the workout, the devices do not allow the user to easily add or remove weight during exercise, the devices are ineffective, the devices are ineffective, or the devices are hard to clean and maintain.

Accordingly, there is a need for an improved device and method for performing Kegel exercises that remedy the shortcomings of the prior art.

### SUMMARY OF INVENTION

According to a first aspect of the present invention there is provided an exercise system as described in claim 1.

According to a second aspect of the present invention there is provided a kit as described in claim 9.

Accordingly, the present invention is directed to an improved device and method for performing male Kegel exercises that provides resistance with an easy-to-use device that is positioned on the shaft proximal to the head of the penis. The device offers a series of magnetic or clip on weights that hang straight down and can be easily added or removed during exercise to adjust weight levels. The design also enables the user to provide assistance to himself during the workout - - a practice known as "spotting" in weightlifting - - which enables the user to create greater pubococcygeus muscle fatigue and improves the effectiveness of the workout. In addition, the design can be easily cleaned and maintained.

The present invention, according to an embodiment, is directed to an exercise system for the pubococcygeus muscles of a male person, the system comprising: an exercise device further comprising: a base and a band coupled to the base; and a supplemental weight coupleable to the base of the exercise device. The band is configurable to fit around the shaft of a penis of a male person so that the base vertically depends from the penis.

The present invention, according to an additional embodiment, is directed to an exercise system for the pubococcygeus muscles of a male person, the system comprising: an exercise device further comprising: a base comprising a magnet and a band coupled to the base; and a supplemental weight comprising a magnet, the supplemental weight being magnetically coupleable to the base of the exercise device. The band is configurable to fit around the shaft of a penis of a male person so that the base vertically depends from the penis.

The magnets in the base and in the supplemental weight are configured so that the supplemental weight will disengage from the base if a predetermined amount of weight is exceeded. The system may further comprise a plurality of supplemental weights, each supplemental weight comprising a magnet; wherein each supplemental weight being is magnetically coupleable to the base and to another supplemental weight. The base may have a tab; each supplemental weight may have a tab; and each supplemental weight may have an indentation configured for receipt of a tab from either the base or another supplemental weight to position each supplemental weight relative to the base and any other supplemental weight.

The present invention, according to an additional embodiment, is directed to an exercise system for the pubococcygeus muscles of a male person, the system comprising: an exercise device further comprising: a base comprising a slot on a bottom side and a band coupled to the base; and a supplemental weight comprising a key on a top side, the supplemental weight key being engageable with the base slot to removeably couple the supplemental weight to the base. The band is configurable to fit around the shaft of a penis of a male person so that the base vertically depends from the penis. The supplemental weight may further comprise a slot on a bottom side for coupling the supplemental weight to an additional supplemental weight.

The present invention, according to an additional embodiment, is directed to an exercise system for the pubococcygeus muscles of a male person, the system comprising: an exercise device further comprising: a base and a band comprising two arms coupled to the base; and a supplemental weight coupleable to the base of the exercise device. The band is configurable to fit around the shaft of a penis of a male person so that the base vertically depends from the penis and to detach from the shaft of a penis of a male person if a predetermined amount of weight is exceeded.

In an embodiment, one of the arms is configured to partially and adjustably nest within the other arm to form a complete ring. In another embodiment, the arms have interlocking portions, the arms being configured so that the interlocking portions are movable relative to each other but still form a closed ring.

The present invention, according to an additional embodiment, is directed to an exercise system for the pubococcygeus muscles of a male person, the system comprising: an exercise device further comprising: a base and a band comprising two arms coupled to the base; and a supplemental weight coupleable to the base of the exercise device. The band is configurable to fit around the shaft of a penis of a male person so that the base vertically depends from the penis. One of the arms has a plurality of holes and the other arm has a pin configured for lockable engagement in one of the holes.

The present invention, according to an additional embodiment, is directed to a kit comprising: an exercise device for the pubococcygeus muscle of a male person, the exercise device comprising: a base; and a band coupled to the base; at least one supplemental weight magnetically coupleable to the exercise device; wherein the band is configurable to fit around the shaft of the penis of a male person so that the base vertically depends from the penis; and wherein the magnets in the base and in the at least one supplemental weight are configured so that the supplemental weight will disengage from the exercise device if a predetermined amount of weight is exceeded. In an additional embodiment, the kit further comprises a plurality of supplemental weights. In an additional embodiment, the kit further comprises a plurality of supplemental weights and a computer readable medium with an exercise program.

The present invention, according to an additional embodiment, is directed to a kit comprising: an exercise device for the pubococcygeus muscle of a male person, the exercise device comprising: a base; and a band comprising two arms coupled to the base; at least one supplemental weight magnetically coupleable to the exercise device; wherein the band is configurable to fit around the shaft of the penis of a male person so that the base vertically depends from the penis; and wherein the magnets in the base and in the at least one supplemental weight are configured so that the supplemental weight will disengage from the exercise device if a predetermined amount of weight is exceeded. One of the arms is configured to partially and adjustably nest within the other arm to form a complete ring; and wherein the band of the support member is configured to detach from the shaft of a penis of a male person if a predetermined amount of weight is exceeded.

### BRIEF DESCRIPTION OF THE DRAWINGS

The features, aspects and advantages of the present invention will become better understood with regard to the following description, appended claims and accompanying figures wherein:
FIG. 1 is a perspective elevation view of an exercise device according to a first embodiment of the present invention;
FIG. 2 is an elevation view of the exercise device of Fig. 1 without supplemental weights;
FIG. 3 is a cross sectional view of the exercise device of Fig. 1;
FIG. 4 is a perspective elevation view of a supplemental weight usable in the exercise device of Fig. 1;
FIG. 5 is a top perspective elevation view of a magnet assembly usable in the exercise device of FIG. 1 and the weight of FIG. 4 according to an embodiment of the present invention;
FIG. 6 is a bottom perspective elevation view of the magnet assembly of FIG. 5;
FIG. 7 is a perspective elevation view of an exercise device according to a second embodiment of the present invention;
FIG. 8 is an elevation view of the exercise device of Fig. 5 without supplemental weights;
FIG. 9 is a perspective elevation view of an exercise device according to a third embodiment of the present invention;
FIG. 10 is an elevation view of the exercise device of Fig. 9;
FIG. 11 is a perspective elevation view of an exercise device according to a fourth embodiment of the present invention;
FIG. 12 is an elevation view of the exercise device of Fig. 11;
FIG. 13 is an elevation view of a weight usable in the exercise device of Fig. 11;
FIG. 14 is a perspective elevation view of an exercise device according to a fifth embodiment of the present invention;
FIG. 15 is an elevation view of the exercise device of Fig. 14 without supplemental weights; and

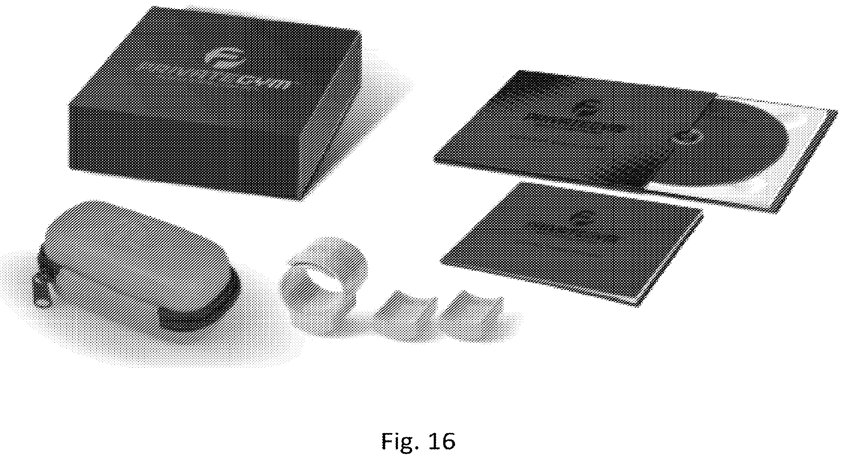

FIG. 16 is a perspective elevation view of an exploded kit containing an exercise device according to an embodiment of the present invention.

### DETAILED DESCRIPTION OF THE PREFERRED EMBODIMENTS

In the following description of the preferred embodiments, reference is made to the accompanying drawings which show by way of illustration specific embodiments in which the invention may be practiced. Wherever possible, the same reference numbers will be used throughout the drawings to refer to the same or like parts. It is to be understood that other embodiments may be utilized and structural and functional changes may be made without departing from the scope of the present invention.

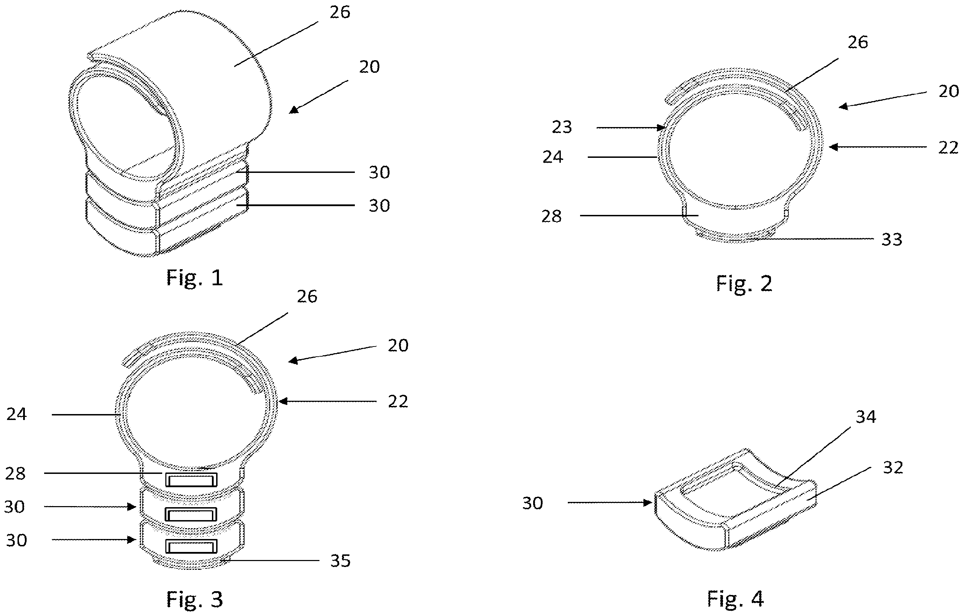

An exercise device according to a first embodiment of the present invention is shown in Figs. 1 to 4. The exercise device 20 has a support member 22. The support member 22 has a band 23 with two arms 24, 26 and a base 28. The arms 24, 26 extend from the base 28 and are precurved to form an adjustable ring configured to fit around the shaft of a male person's penis. In the first embodiment, one of the arms is configured to partially and adjustably nest within the other arm to form a complete ring around the penis. In an alternative embodiment, the support member 22 may be formed of a single curved arm.

Preferably the arms 24, 26 are made of molded rubber or pliable plastic, such that the arms can be moved relative to one another allow the device to fit around differently sized penises, yet are rigid enough that the arms cannot be bent under a predetermined weight to completely disengage from the shaft of the user's penis. Preferably, the arms 24, 26 are configured disengage from a user's penis if the user attempts to increase the total weight beyond the predetermined weight. In an embodiment, the arms 24, 26 are made of a thermoplastic elastomer formed over spring steel.

The base 28 is coupled to the arms 24, 26. Preferably, the base 28 is made of rubber or plastic and is molded at the same time as the arms. However, the base 28 may be made of different material than the arms and may be coupled to the arms. In an embodiment, a weight is molded into the base 28. The weight may be made from any reasonably dense material. In an embodiment, the weight is made from metal, such as steel, iron or lead. The weight can be molded into the base or coupled to the base. The weight may weigh from about 1 ounce to about 5 ounces and more preferably from about 2 ounces to about 3 ounces.

In an embodiment, the exercise device 20 further comprises at least one supplemental weight 30 coupleable to the base 28. The supplemental weight 30 may include a weight made from the same material as the base weight. Alternatively, the supplemental weights 30 can be made from a different material than the base weight. Preferably, the weight source is molded into a plastic or rubber casing 32 that matches the material of the support member 12.

Preferably, as shown in Figs. 1 and 3, the exercise device 20 has a plurality of supplemental weights 30 that are attachable to each other and to the base 28. In an embodiment, the supplemental weights 30 each have the same weight. In an alternative embodiment, the supplemental weights 30 have different weights. Each supplemental weight may weigh from about 1 ounce to about 6 ounces and more preferably from about 2 ounces to about 4 ounces.

In the embodiment shown in Figs. 1 to 6 the weights in the base 28 and in the supplemental weights 30 are magnetic. The supplemental weights 30 may be magnetically coupled to each other and to the base 28. Preferably, the base 28 has a tab 33 on a bottom side and the supplemental weight 30 has an indentation 34 on a top side each such that the base tab 33 may engage with the indentation 34 to fit the supplemental weight 30 on the base.

The engagement of the tab 33 and the indentation 34 preferably allows for optimum positioning of the magnetic base weight and magnetic supplemental weight with one hand and without the need to view the connection. Preferably, each supplemental weight 30 has a tab 35 on a bottom side for engagement with the indentation 34 of another supplemental weight 30 to allow multiple supplemental weights to be fitted to the base 28. As will be understood by one of skill in the art, the configuration of the tab and the indentation may be reversed such that the base has an indentation, and the supplemental weight has a tab on a top side and an indentation on a bottom side.

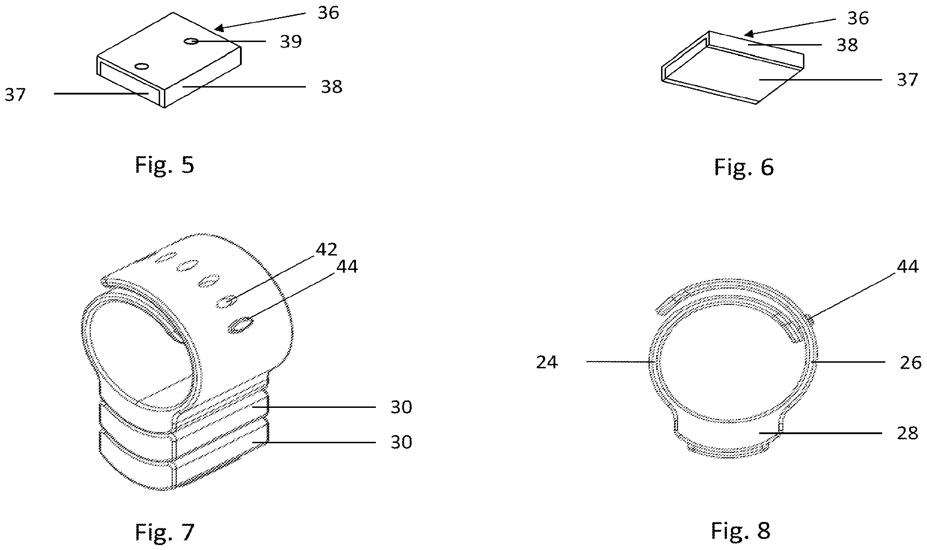

A magnetic weight 36 according to an embodiment of the present invention is shown in Figs. 5 and 6. The weight 36 is formed by coupling a magnet 37 to a metal jacket 38. The magnet 37 may be coupled to the metal jacket 38 using epoxy. Additionally, the magnet 37 may have one or more tabs (not shown) that fit inside of one or more holes 39 in the jacket 38 to position the magnet inside of the jacket. The weights 36 are molded into the base 28 of the exercise device 20 and into each of the supplemental weights 30. The dimensions of the metal jacket 38 may be selected depending on the desired weight. The dimensions and magnetic strength of the magnet 37 may be selected depending on the desired attraction between the device 20 and supplemental weights 30 as further discussed below.

The magnetic weights 36 in the base 28 and the supplemental weights 30 are configured to magnetically couple the supplemental weights to the base so that the supplemental weights will not disengage from the base during normal use. The magnets 36 in the base 28 and the supplemental weights 30 are further configured so that the supplemental weights will disengage from the base if the user attempts to use more than a predetermined amount of supplemental weight. Preferably, the magnets 36 in the base 28 and the supplemental weights 30 are further configured to allow a user to easily vary the number of supplemental weights used.

The magnetic weights 36 in the base 28 and the supplemental weights 30 are used in conjunction with the base tab 33, supplemental weight indentations 34 and supplemental weight tabs 35 so that a user may easily position and detach supplemental weights with one hand and without looking at the device. To use a supplemental weight 30 with the device, a user positions the indentation 34 over the base tab 33 and thereafter the magnetic weights 36 in the base 28 and the supplemental weight removeably couple the supplemental weight to the base. If the user wishes to add another supplemental weight 30, then the user positions the additional supplemental weight indentation 34 over the previously added supplemental weight tab 35. Thereafter, the magnetic weights 36 in the supplemental weights 30 hold the supplemental weights together. If the user wishes to remove a supplemental weight 30, then the user may pull the supplemental weight away from the base 28 or adjacent supplemental weight.

A second embodiment of the present invention is shown in Figs. 7 and 8. In the second embodiment one of the arms has a plurality of holes 40 along its length and the other arm has a pin 42 configured for lockable engagement in one of the holes 40. The holes 40 and the pin 42 allows for a user to set the size of the ring formed by the arms. This may allow for a more comfortable fit and may also allow for the use of less resilient materials for the arms. The number of holes and pins may be varied.

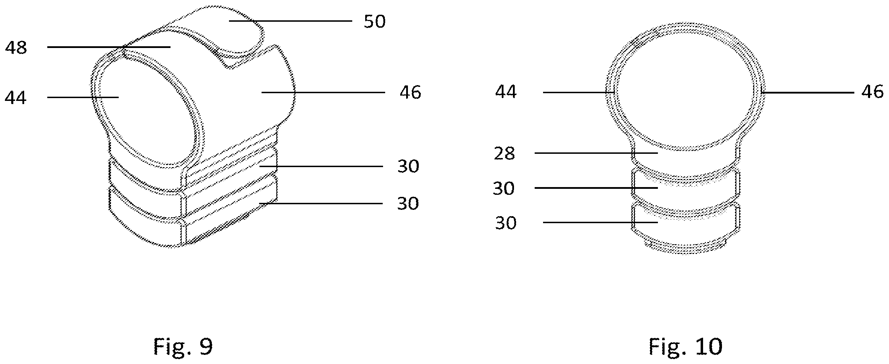

A third embodiment of the present invention is shown in Figs. 9 and 10. In the third embodiment, as in the first embodiment, the exercise device 20 has a support member 22 and a base 28. The support member 22 has two arms 44, 46 that extend from the base 28 and are precurved to form an adjustable ring configured to fit around the shaft of a male person's penis. The arms 44, 46 are pliable and have interlocking portions 48, 50 that can be moved relative to one another to accommodate differently sized penises, yet together still form a closed ring to prevent the device from completely disengaging from the shaft of the user's penis during exercises when weights are used.

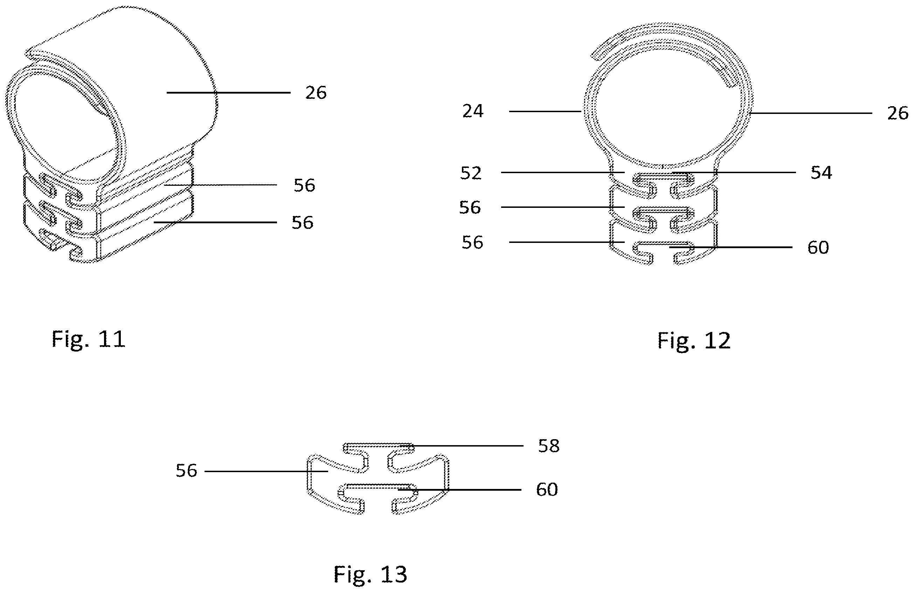

A fourth embodiment of the present invention is shown in Figs. 10 to 12. The fourth embodiment contains arms as previously described with regard to the first embodiment shown in Figs. 1 to 4. However, the support member 22 has a differently configured base 52. The base has a t-shaped slot 54 extending longitudinally along a bottom side. The supplemental weight 56 has a t-shaped key 58 extending longitudinally along a top side configured such that the supplemental weight key 58 may engage with the base slot 54 to securely couple the supplemental weight 56 to the base 52. Preferably, as shown in Fig. 11, each supplemental weight has a t-shaped slot 60 on a bottom side for engagement with the key 58 of another supplemental weight 56 to allow multiple supplemental weights to be fitted to the base 52.

The use of a key and slot mechanism for attaching the supplemental weights 56 to the base 52 allows for the use of non-magnetized bases and supplemental weights. Preferably, the outer covering of the base 52 and the supplemental weights has a high enough coefficient of static friction to prevent movement of the supplemental weights 56 relative to the base 52 during normal exercise usage.

As will be understood by one of skill in the art, the configuration of the key and the slot may be reversed such that the base has a key extending longitudinally along a bottom side, the supplemental weight has a key shaped slot extending longitudinally along a top side. Additionally, the supplemental weight may have a key extending longitudinally along a bottom side for engagement with the slot of another supplemental weight to allow multiple supplemental weights to be fitted to the base.

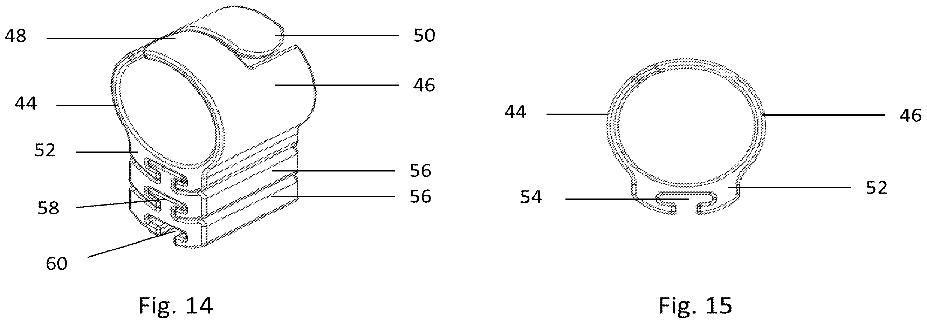

A fifth embodiment of the present invention is shown in Figs. 14 and 15. The fifth embodiment contains arms 44, 46 with overlapping portions 48, 50 as previously described with regard to the third embodiment shown in Figs. 7 and 8. The fifth embodiment further contains a base 52 having a t-shaped slot 54 and supplemental weights 56 having a t-shaped key 58 and a t-shaped slot 60 as described with regard to the fourth embodiment shown in Figs. 9 to 11.

A method of using an exercise device in accordance with the present invention will now be described. A user is provided with an exercise device comprising a support member with two arms and a base. The arms of the support member are manipulated to securely place the exercise device on an erect penis of the male person with the base depending in a vertical manner from the penis. The user may then add or remove supplemental weights attached to the vertically depending base or "spot" themselves during their exercises. The user then performs Kegel exercises with the exercise device placed on the penis. Preferably, the exercise device is placed such that the support member is retained on the shaft of the penis proximal to the head of the penis.

In an additional embodiment, the user selects at least one supplemental weight and attaches the supplemental weight to the base of the exercise device. The at least one supplemental weight may be selected from a plurality of different supplemental weights. A plurality of supplemental weights may be attached with a first supplemental weight being attached to the base and additional supplemental weights attached to the last already attached supplemental weight.

The present invention is also directed to a kit containing the exercise device 20 and at least one supplemental weight 30. In an additional embodiment, the kit has the exercise device 20 and at least two supplemental weights 30. In an additional embodiment, the kit has the exercise device 20, at least two supplemental weights 30 and a carrying case. In an additional embodiment, the kit has the exercise device 20, at least two supplemental weights 30, a carrying case and a computer readable medium, such as a DVD, with an exercise program for a user.

There is disclosed in the above description and the drawings, an exercise device which overcomes the disadvantages associated with the prior art. However, it will be apparent that variations and modifications of the disclosed embodiments may be made without departing from the principles of the invention. The presentation of the preferred embodiments herein is offered by way of example only and not limitation, with a true scope of the invention being indicated by the following claims.

## Claims

1. An exercise system for the pubococcygeus muscles of a male person, the system comprising:
an exercise device further comprising: a base and a band coupled to the base, said band including two arms wherein one of said two arms is configured to partially and adjustably nest with the other arm to form a complete ring; and
a supplemental weight coupleable to the base of the exercise device;
wherein the band is configurable to fit around the shaft of a penis of a male person so that the base vertically depends from the penis,
**characterised in that** the base further comprises a magnet, the supplemental weight further comprises a magnet; the supplemental weight is magnetically coupleable to the base; and wherein the magnets in the base and in the supplemental weight are configured so that the supplemental weight will disengage from the base if a predetermined amount of weight is exceeded, thereby enabling an user to provide assistance to himself during the workout, a practice known as spotting in weightlifting.

2. The exercise system of claim 1 further comprising a plurality of supplemental weights, each supplemental weight comprising a magnet; wherein each supplemental weight is magnetically coupleable to the base and to another supplemental weight.

3. The exercise system of claim 2 wherein the base further comprises a tab; each supplemental weight further comprises a tab; and wherein each supplemental weight comprises an indentation configured for receipt of a tab from either the base or another supplemental weight to position each supplemental weight relative to the base and any other supplemental weight.

4. The exercise system of claim 1 wherein the base further comprises a slot on a bottom side, the supplemental weight further comprises a key on a top side; and wherein the supplemental weight key is engageable with the base slot for removeably coupling the supplemental weight to the base.

5. The exercise system of claim 4 wherein the supplemental weight further comprises a slot on a bottom side for coupling the supplemental weight to an additional supplemental weight.

6. The exercise system of claim 1 wherein the band is configured to detach from the shaft of a penis of a male person if a predetermined amount of weight is exceeded.

7. The exercise system of claim 6 wherein the arms have interlocking portions, the arms being configured so that the interlocking portions are movable relative to each other but still form a closed ring.

8. The exercise system of claim 1 wherein one of the arms has a plurality of holes and the other arm has a pin configured for lockable engagement in one of the holes.

9. A kit comprising an exercise system according to claim 1 and further comprising a plurality of supplemental weights.

10. The kit of claim 9 further comprising a computer readable medium with an exercise program.

11. The kit of claim 10 wherein the band is configured to detach from the shaft of a penis of a male person if a predetermined amount of weight is exceeded.

## Patentansprüche

1. Ein Trainingssystem für den Pubococcygeus Muskel von männlichen Personen, das System umfassend:
ein Trainingsgerät, ferner umfassend: eine Basis und einen Ring verbunden mit der Basis, wobei jener Ring zwei Arme einschließt, worin einer der beiden Arme konfiguriert ist, um zur Bildung eines kompletten Ringes teilweise und anpassbar mit dem anderen Arm ineinanderzugreifen; und
ein zusätzliches Gewicht, das mit der Basis des Trainingsgeräts verbunden werden kann; worin der Ring konfigurierbar ist, um den Schaft des Penis einer männlichen Person herumzupassen, so dass die Basis vertikal vom Penis herunterhängt,
dadurch charakterisiert, dass die Basis ferner einen Magnet umfasst, das zusätzliche Gewicht einen Magnet umfasst; das zusätzliche Gewicht magnetisch mit der Basis verbunden werden kann; und worin die Magnete in der Basis und im zusätzlichen Gewicht so konfiguriert sind, dass das zusätzliche Gewicht sich von der Basis lösen wird, wenn eine vordefinierte Menge an Gewicht überschritten wird, wobei es dem Verwender ermöglicht wird, sich selbst während des Trainings zu assistieren, eine Praxis, die vom Gewichtheben als Spotting bekannt ist.

2. Das Trainingssystem nach Anspruch 1, ferner umfassend eine Vielzahl an zusätzlichen Gewichten, wobei jedes zusätzliche Gewicht einen Magnet umfasst; worin jedes zusätzliche Gewicht magnetisch mit der Basis und mit einem anderen zusätzlichen Gewicht verbunden werden kann.

3. Das Trainingssystem nach Anspruch 2, worin die Basis ferner einen Aufhänger umfasst; jedes zusätzliche Gewicht ferner einen Aufhänger umfasst; und worin jedes zusätzliche Gewicht eine Vertiefung umfasst, die für die Aufnahme eines Aufhängers entweder von der Basis oder einem anderen zusätzlichen Gewicht konfiguriert ist, um jedes zusätzliche Gewicht relativ zur Basis und jedem anderen zusätzlichen Gewicht zu positionieren.

4. Das Trainingssystem nach Anspruch 1, worin die Basis ferner einen Schlitz auf der Unterseite umfasst, das zusätzliche Gewicht ferner einen Schlüssel auf der Oberseite umfasst; und worin der Schlüssel des zusätzlichen Gewichts mit dem Schlitz der Basis verbunden werden kann, zum abnehmbaren Verbinden des zusätzlichen Gewichts mit der Basis.

5. Das Trainingssystem nach Anspruch 4, worin das zusätzliche Gewicht ferner einen Schlitz auf der Unterseite umfasst, um das zusätzliche Gewicht mit einem weiteren zusätzlichen Gewicht zu verbinden.

6. Das Trainingssystem nach Anspruch 1, worin der Ring konfiguriert ist, um sich vom Schaft des Penis einer männlichen Person zu lösen wenn eine vordefinierte Menge an Gewicht überschritten wird.

7. Das Trainingssystem nach Anspruch 6, worin die Arme ineinandergreifende Teile haben, wobei die Arme so konfiguriert sind, dass die ineinandergreifenden Teile relativ zueinander bewegbar sind, aber noch einen geschlossenen Ring bilden.

8. Das Trainingssystem nach Anspruch 1, worin einer der Arme eine Vielzahl von Löchern und der andere Arm einen Pin hat, der für die feststellbare Verbindung in einem der Löcher konfiguriert ist.

9. Ein Kit, umfassend ein Trainingssystem nach Anspruch 1 und ferner umfassend eine Vielzahl von zusätzlichen Gewichten.

10. Das Kit nach Anspruch 9, ferner umfassend ein Computer-lesbares Medium mit einem Trainingsprogramm.

11. Das Kit nach Anspruch 10, worin der Ring konfiguriert ist, um sich vom Schaft des Penis einer männlichen Person zu lösen, wenn eine vordefinierte Menge an Gewicht überschritten wird.

## Revendications

1. Système d'exercice pour les muscles pubo-coccygiens d'un homme, le système comprenant :
un dispositif d'exercice comprenant en outre : une base et une bande couplée à la base, ladite bande comprenant deux bras, l'un desdits deux bras étant configuré pour s'emboîter de manière partielle et ajustable avec l'autre bras pour former un anneau entier ; et
un poids supplémentaire apte à être couplé à la base du dispositif d'exercice ;
la bande étant configurable pour s'ajuster autour du corps d'un pénis d'un homme de telle sorte que la base pend verticalement à partir du pénis,
**caractérisé par le fait que** la base comprend en outre un aimant, le poids supplémentaire comprend en outre un aimant ; le poids supplémentaire étant apte à être couplé magnétiquement à la base ; et les aimants dans la base et dans le poids supplémentaire étant configurés de telle sorte que le poids supplémentaire se désengagera de la base si une quantité de poids prédéterminée est dépassée, permettant ainsi à un utilisateur de se venir en aide lui-même pendant l'entraînement, une pratique connue sous la dénomination de soutien en haltérophilie.

2. Système d'exercice selon la revendication 1, comprenant en outre une pluralité de poids supplémentaires, chaque poids supplémentaire comprenant un aimant ; chaque poids supplémentaire étant apte à être couplé magnétiquement à la base et à un autre poids supplémentaire.

3. Système d'exercice selon la revendication 2, dans lequel la base comprend en outre une languette ; chaque poids supplémentaire comprend en outre une languette ; et chaque poids supplémentaire comprend une encoche configurée pour recevoir une languette à partir soit de la base soit d'un autre poids supplémentaire pour positionner chaque poids supplémentaire par rapport à la base et tout autre poids supplémentaire.

4. Système d'exercice selon la revendication 1, dans lequel la base comprend en outre une fente sur un côté inférieur, le poids supplémentaire comprend en outre une clavette sur un côté supérieur ; et la clavette de poids supplémentaire est apte à s'engager avec la fente de base pour accoupler le poids supplémentaire à la base de manière amovible.

5. Système d'exercice selon la revendication 4, dans lequel le poids supplémentaire comprend en outre une fente sur un côté inférieur pour accoupler le poids supplémentaire à un poids supplémentaire additionnel.

6. Système d'exercice selon la revendication 1, dans lequel la bande est configurée pour se détacher du corps d'un pénis d'un homme si une quantité de poids prédéterminée est dépassée.

7. Système d'exercice selon la revendication 6, dans lequel les bras ont des parties de verrouillage mutuel, les bras étant configurés de telle sorte que les parties de verrouillage mutuel sont mobiles l'une par rapport à l'autre, mais forment toujours un anneau fermé.

8. Système d'exercice selon la revendication 1, dans lequel l'un des bras a une pluralité de trous et l'autre bras a une broche configurée pour un engagement verrouillable dans l'un des trous.

9. Kit comprenant un système d'exercice selon la revendication 1 et comprenant en outre une pluralité de poids supplémentaires.

10. Kit selon la revendication 9, comprenant en outre un support lisible par ordinateur ayant un programme d'exercice.

11. Kit selon la revendication 10, dans lequel la bande est configurée pour se détacher du corps d'un pénis d'un homme si une quantité de poids prédéterminée est dépassée.
